# EUROPEAN PATENT APPLICATION

(11) **EP 3 349 218 A1**
(43) Date of publication of application: **18.07.2018**
(21) Application number: 17193529.9
(22) Date of filing: 27.09.2017
(51) Int. Cl.: G16H 30/40

(54) **METHOD AND SYSTEM FOR CONFIGURING A MEDICAL DATASET PROCESSING WORKFLOW**

(71) Applicant: Siemens Healthcare GmbH, 91052 Erlangen (DE)
(72) Inventor: Battle, Xavier, 91301 Forchheim (DE); Schmidt, Bernhard, 90766 Fürth (DE); Schmidt, Sebastian, 91085 Weisendorf (DE); Soza, Grzegorz, 90562 Heroldsberg (DE)

(57) **Abstract**

The invention relates in one aspect to a method for configuring a medical dataset processing workflow, the method comprising:
- providing a training precondition for a training of a machine learning algorithm,
- selecting a first training pair for the training of the machine learning algorithm based on the training precondition, the first training pair comprising a medical dataset and a processing result of a first processing of the medical dataset,
- determining a processing precondition for a planned second processing of the medical dataset based on the training precondition and the first training pair,
- configuring a worklist of the medical dataset processing workflow based on the processing precondition, the configured worklist comprising a task regarding the planned second processing of the medical dataset.

## Description

The invention relates in one aspect to a method for configuring a medical dataset processing workflow. In another aspect, the invention relates to a system for configuring a medical dataset processing workflow. In further aspects, the invention relates to a computer program product and a computer-readable medium.

Modern image and information processing systems are based on technologies like machine learning. Such systems rely on the underlying training examples. In a supervised training scenario, such training examples would be usually generated by a domain expert. In the field of medical dataset, reading domain experts are, for example, radiologists, clinicians, technicians or other clinical users. These training examples are then fed to the algorithm, which analyzes the training data and learns respective image features and/or characteristics, statistical correlations, etc. Finally, such systems can generalize from the training data to unseen situations and provide an automatic solution for unseen cases.

The creation of training examples usually requires intensive manual work. Such effort is usually spent in dedicated sessions with domain experts and requires huge organizational and financial effort. The content of training examples is very often subjective and depends on the expert who created it. For example, training examples containing liver segmentation masks can be used for a supervised machine learning algorithm which then, after training phase, can automatically segment liver from anatomical scans on unseen data. Such training segmentation masks would be created by expert radiologists. It is obvious that liver segmentation masks created by two different clinical users for the same anatomical scan will never be exactly the same. There will be always some variation due to subjective interpretation of human, due to imprecise delineation of contours with the available tool, due to the level of experience, etc. Even if one and the same clinical user would process the case twice, it is expected that these two results would differ.

The underlying technical problem of the invention is to facilitate an improved collection of training data for machine learning. This problem is solved by the method of claim 1, by the system of claim 10, by the computer program product of claim 14 and by the computer-readable medium 15. The dependent claims are related to further aspects of the invention.

The invention relates in one aspect to a method for configuring a medical dataset processing workflow, the method comprising:
- providing a training precondition for a training of a machine learning algorithm,
- selecting a first training pair for the training of the machine learning algorithm based on the training precondition, the first training pair comprising a medical dataset and a processing result of a first processing of the medical dataset,
- determining a processing precondition for a planned second processing of the medical dataset based on the training precondition and the first training pair,
- configuring a worklist of the medical dataset processing workflow based on the processing precondition, thereby obtaining a configured worklist, the configured worklist comprising a task regarding the planned second processing of the medical dataset.

The medical dataset may comprise medical data of the patient, for example a medical image and/or diagnostic data and/or clinical data, in particular regarding the medical history of the patient. The medical dataset may comprise patient data, in particular regarding demographical, physiological and/or ethnical aspects of the patient. Patient data may comprise, for example, information regarding the weight and/or the age of the patient.

The invention relates in one aspect to a method for configuring a medical image processing workflow, the method comprising:
- providing a training precondition for a training of a machine learning algorithm,
- selecting a first training pair for the training of the machine learning algorithm based on the training precondition, the first training pair comprising a medical image and a processing result of a first processing of the medical image,
- determining a processing precondition for a planned second processing of the medical image based on the training precondition and the first training pair,
- configuring a worklist of the medical image processing workflow based on the processing precondition, thereby obtaining a configured worklist, the configured worklist comprising a task regarding the planned second processing of the medical image.

The training precondition may comprise, for example, a requirement regarding the type of training examples that is needed in order to train the machine learning algorithm. For example, vessel centerlines are needed to train a centerline tracing algorithm, liver masks are needed to train a liver volumetrization algorithm and tumor masks are needed to train a tumor volumetrization algorithm. The training precondition may comprise, for example, a requirement regarding a number of processing results to be obtained for the same medical image. For example, the training precondition may comprise a requirement that a certain number of processing results, e.g. three processing results, per medical image are needed. Such a requirement may be determined based on an expected variation in the processing results. Information regarding an expected variation in the processing results may be provided to the system "a priori". Such information may be based on an evaluation by a user and/or on empirical values of variation in the processing results of comparable cases.

The training precondition, in particular the requirement regarding a number of processing results to be obtained for the same medical image, may be adjusted based on an recently obtained processing result, in particular the processing result of the first processing of the medical image.

For example the training precondition may comprise a requirement, that an initial number of processing results per medical image is needed. The initial number may be determined, for example, based on empirical values of variation in the processing results of comparable cases. The performance of the trained machine learning algorithm may be monitored based, for example, on a quality measure of the machine learning algorithm output. This monitoring may be executed continuously, for example, based on a predefined time interval and/or a predefined number of recently obtained training pair. If the performance improves, a higher number of processing results per medical image would provide additional benefit. If the performance converges, there is no need to further increase the number of processing results per medical image. The case, that performance actually decreases, would be something not expected. It would rather be an indication that a bias has been introduced to the training data.

In one embodiment, the processing condition comprises
- a requirement regarding a time interval between the first processing of the medical image and the planned second processing of the medical image and/or
- a requirement regarding a user, to whom the task regarding the planned second processing of the medical image may be assigned, in particular a user, to whom the task regarding the planned second processing of the medical image is assigned.

The processing condition may comprise, for example, a requirement that a user to whom the first processing of the medical image has been assigned is not allowed to control the planned second processing of the medical image unless a certain time interval after the first processing of the medical image has expired. The processing condition may comprise, for example, a requirement that the planned second processing of the medical image has to be assigned to a user to whom the first processing of the medical image has not been assigned and/or that the planned second processing has to be performed in another clinical environment, e.g. hospital.

In one embodiment, a graphical element indicative of the task regarding the planned second processing is displayed in a graphical user interface with regard to the processing condition. The configured worklist, in particular the task regarding the planned second processing, may be stored in a memory for further processing.

The graphical element indicative of the task regarding the planned second processing may be displayed, for example, to a user to whom the first processing of the medical image has been assigned only after a certain time interval after the first processing of the medical image has expired and/or only to a user to whom the first processing of the medical image has not been assigned. A list of users may be provided. Based on the processing requirement, one or more users may be selected from the list to whom the task regarding the planned second processing of the medical image may be or may not be assigned.

The graphical element may comprise, for example, a label indicative of the processing condition. The label may comprise, for example, a date information and/or a time information indicative of at least a beginning, an end and/or a duration of the time interval between the first processing of the medical image and the planned second processing of the medical image. The label may comprise, for example, a user identity information indicative of at least a user to whom the task regarding the planned second processing of the medical image may be assigned and/or a user to whom the task regarding the planned second processing of the medical image may not be assigned.

In one embodiment, a user input in order to select the task regarding the planned second processing is received, e.g. through the graphical element. A check with regard to the processing condition may be performed upon receiving the user input. Based on the outcome of the check, the task regarding the planned second processing of the medical image may be assigned to the user and/or the user to whom the task is assigned may control the planned second processing, thereby obtaining a result of the second processing. A user may control a processing of the medical image, in particular the first processing and/or the second processing of the medical image, manually and/or semi-manually. For example, the user may semi-manually adjust an automatically generated segmentation mask or manually delineate a centerline.

In one embodiment, the planned second processing of the medical image is performed, e.g. with regard to the processing condition, thereby obtaining a second training pair for the training of the machine learning algorithm, the second training pair comprising the medical image and a processing result of the planned second processing of the medical image.

In one embodiment, the machine learning algorithm is trained based on a set of training pairs, the set of training pairs comprising the first training pair and the second training pair. In one embodiment, an uncertainty information is determined based on the first training pair and the second training pair. The machine learning algorithm may be trained based on the uncertainty information.

The uncertainty information may comprise, for example, a probability mask. The probability mask may be determined, for example, based on the processing result of the first processing and the processing result of the planned second processing. In particular, the processing result of the first processing result may comprise a first binary mask and the processing result of the planned second processing may comprise a second binary mask. A binary mask may assign, for example, to each pixel of the medical image one value out of two values. For example, one value, in particular 1 or True, may be assigned to the pixels representing an anatomical structure or a contour or a centerline of the anatomical structure in the medical image according to a given processing result and the other value, in particular 0 or False, may be assigned to the remaining pixels of the medical image.

The probability mask may assign to each pixel of the medical image a value corresponding to a value in between the two values used in the binary masks. The value assigned to a given pixel in the medical image according to the probability mask may correlate with the probability, that the given pixel is in fact representing the anatomical structure or a contour or a centerline of the anatomical structure. For example, if two different contours delineating the same lesion are obtained, this information can be used to say that any contours "between" those two contours are with some probability a valid input. A further training pair may be obtained, comprising the medical image as a training input and the probability mask as a training output.

The uncertainty information may comprise, for example, a weighting information, assigning to the medical image a weighting value indicative of a degree of variation of the processing results obtained based on the medical image. For example, medical images, for which processing results show a rather low degree of variation, and medical images for which processing results show a rather high degree of variation, may be used in different ways for training the machine learning algorithm.

In one embodiment, the medical image processing workflow is a clinical workflow and/or is performed in a clinical environment, e.g. a hospital. The medical image processing workflow may be a radiology reading workflow and/or a surgery planning workflow. In particular, the first processing of the medical image may be a first reading of the medical image and the planned second processing of the medical image may be a planned second reading of the medical image.

In one embodiment, the processing result of the first processing of the medical image is a first segmentation mask of an anatomical structure in the medical image and the processing result of the planned second processing of the medical image is a second segmentation mask of the anatomical structure in the medical image. In one embodiment, the processing result of the first processing of the medical image is a first centerline delineation of an anatomical structure in the medical image and the processing result of the planned second processing of the medical image is a second centerline delineation of the anatomical structure in the medical image.

The invention relates in one aspect to a system for configuring a medical dataset processing workflow, the system comprising:
- a training-precondition-providing unit configured for providing a training precondition for a training of a machine learning algorithm,
- a first training-pair-selecting unit configured for selecting a first training pair for the training of the machine learning algorithm based on the training precondition, the first training pair comprising a medical dataset and a processing result of a first processing of the medical dataset,
- a processing-precondition-determining unit configured for determining a processing precondition for a planned second processing of the medical dataset based on the training precondition and the first training pair,
- a worklist-configuring unit configured for configuring a worklist of the medical dataset processing workflow based on the processing precondition, the configured worklist comprising a task regarding the planned second processing of the medical dataset.

The invention relates in one aspect to a system for configuring a medical image processing workflow, the system comprising:
- a training-precondition-providing unit configured for providing a training precondition for a training of a machine learning algorithm,
- a first training-pair-selecting unit configured for selecting a first training pair for the training of the machine learning algorithm based on the training precondition, the first training pair comprising a medical image and a processing result of a first processing of the medical image,
- a processing-precondition-determining unit configured for determining a processing precondition for a planned second processing of the medical image based on the training precondition and the first training pair,
- a worklist-configuring unit configured for configuring a worklist of the medical image processing workflow based on the processing precondition, the configured worklist comprising a task regarding the planned second processing of the medical image.

In one embodiment, the system further comprises a training unit configured for the training of the machine learning algorithm, e.g. based on a set of training pairs, and/or a medical image processing unit configured for performing the planned second processing of the medical image.

In one embodiment, the medical image processing unit is located inside a clinical environment and/or the training unit is located external to the clinical environment. The system may further comprise a connection between the image processing unit and the training unit to facilitate data exchange between the medical image processing unit and the training unit.

In one embodiment, the system is configured for performing a method according to one or more of the aspects disclosed in this application.

The invention relates in one aspect to a computer program product including a computer program, directly loadable into a memory device of a computer, including program sections for carrying out a method according to one or more of the aspects disclosed in this application, when the computer program is executed in said computer.

The invention relates in one aspect to a computer-readable medium storing program sections, readable and executable by a computer, to carry out a method according to one or more of the aspects disclosed in this application, when the program sections are executed by the computer.

The system for configuring a medical dataset and/or medical image processing workflow can be realized as a data processing system or as a part of a data processing system. The data processing system can, for example, comprise a cloud-computing system, a computer network, a computer, a tablet computer, a smartphone or the like. The data processing system can comprise hardware and/or software. The hardware can be, for example, a processor system, a memory system and combinations thereof. The hardware can be configurable by the software and/or be operable by the software. Calculations, for example for training of the machine learning algorithm and/or for determining of a processing condition and/or for performing other steps of the method may be carried out, for example, in a processor.

The machine learning algorithm may be implemented, for example, based on decision trees, deep learning, reinforcement learning, marginal space learning, a support vector machine, an artificial neural network, in particular a convolutional neural network or a neural network-based regression function. A convolutional neural network can be configured for selecting, based on the training data, a member function of a class of functions based on adaptive weigths tuned by a learning algorithm.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The medical image may be obtained by a medical imaging device and/or retrieved from a medical image database. The medical imaging device can be, for example, be selected from the group consisting of a computed tomography device, a magnetic resonance imaging device, a molecular imaging device, a SPECT-device, a PET-device and combinations thereof. The medical imaging device can be, for example, a combination of an imaging modality and a therapy modality, in particular a radiation therapy modality.

Reference is made to the fact that the described methods and the described system are merely preferred example embodiments of the invention and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
Fig. 1 shows a diagram illustrating a method for configuring a medical image processing workflow.
Fig. 2 shows a system for configuring a medical image processing workflow.
Fig. 3 shows a workstation for handling tasks of a medical imaging processing workflow.
Fig. 4 shows a network comprising a development system and several workstations.
Fig. 5 shows examples of different processing results for the same medical image.
Fig. 6 shows examples of different processing results obtained using different workstations.

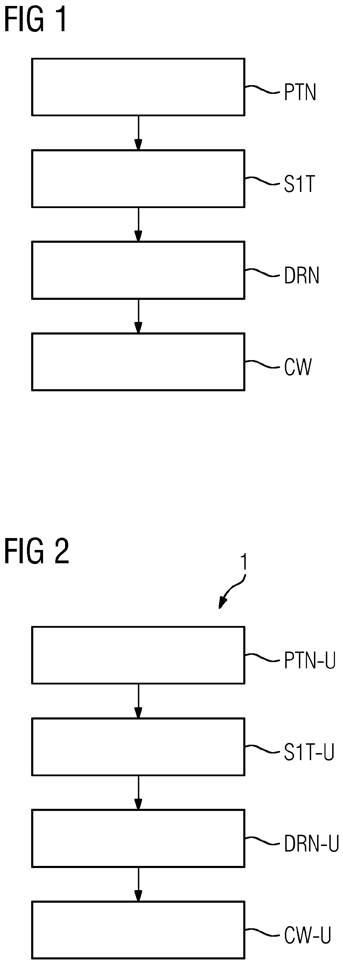

Fig. 1 shows a diagram illustrating a method for configuring a medical image processing workflow, the method comprising:
- providing PTN a training precondition for a training of a machine learning algorithm,
- selecting S1T a first training pair for the training of the machine learning algorithm based on the training precondition, the first training pair comprising a medical image MI and a processing result of a first processing of the medical image MI,
- determining DRN a processing precondition for a planned second processing of the medical image MI based on the training precondition and the first training pair,
- configuring CW a worklist of the medical image processing workflow based on the processing precondition, the configured worklist comprising a task regarding the planned second processing of the medical image.

Fig. 2 shows a system 1 for configuring a medical image processing workflow, the system comprising:
- a training-precondition-providing unit PTN-U configured for providing PTN a training precondition for a training of a machine learning algorithm,
- a first training-pair-selecting unit S1T-U configured for selecting S1T a first training pair for the training of the machine learning algorithm based on the training precondition, the first training pair comprising a medical image MI and a processing result of a first processing of the medical image MI,
- a processing-precondition-determining unit DRN-U configured for determining DRN a processing precondition for a planned second processing of the medical image MI based on the training precondition and the first training pair,
- a worklist-configuring unit CW-U configured for configuring CW a worklist of the medical image processing workflow based on the processing precondition, the configured worklist comprising a task regarding the planned second processing of the medical image.

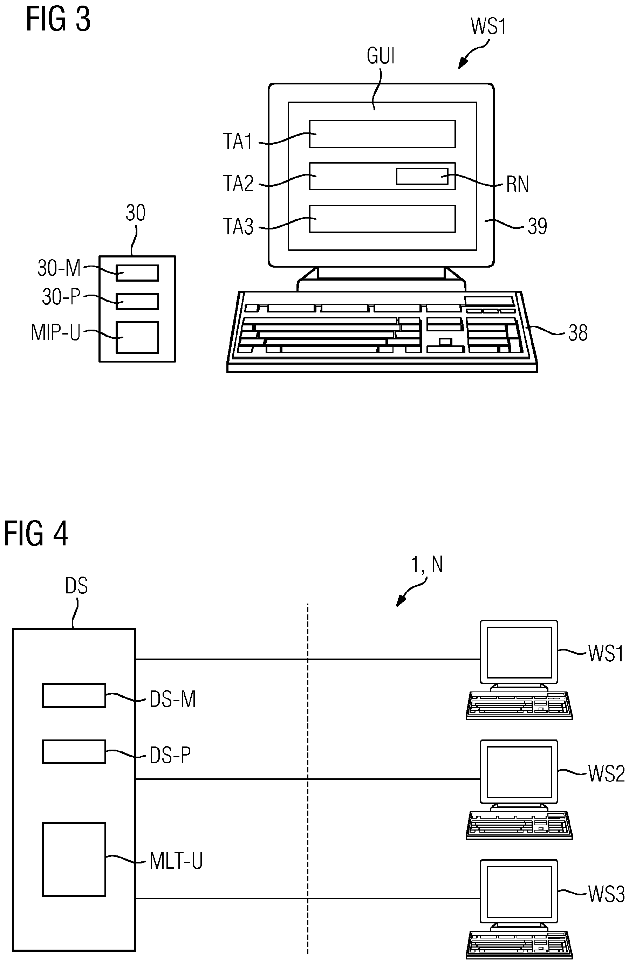

Fig. 3 shows a workstation WS1 for handling tasks of a medical imaging processing workflow. The workstation WS1 comprises a control device 30 for controlling the workstation WS1. The control device 30 comprises a computer-readable medium 30-M and a processor 30-P. The control device 30 may be, for example, a computer and/or at least a part of a data processing system. The workstation WS1 further comprises an input unit 38 for inputting a user input, for example a user input in order to select the task regarding the planned second processing and/or a user input in order to manually and/or semi-manually control the second processing of the medical image MI. The workstation WS1 further comprises an output unit 39 for outputting information and images, in particular the graphical user interface GUI, the medical image MI, the processing result L1 of the first processing of the medical image MI, the processing result L2 of the planned second processing of the medical image MI. In the graphical user interface GUI, graphical elements TA1, TA2, TA3 are displayed, each of the graphical elements TA1, TA2, TA3 being indicative of a corresponding task of a worklist of the medical image processing workflow. The graphical element TA2 is indicative of the task regarding the planned second processing and comprises a label RN indicate of the processing condition. The control device further comprises a medical image processing unit MIP-U configured for performing the planned second processing of the medical image MI.

Fig. 4 shows a network N comprising a development system DS and several workstations WS1, WS2, WS3. The development system comprises a training unit MLT-U configured for the training of the machine learning algorithm based on a set of training pairs. The development system DS comprises a computer-readable medium DS-M and a processor DS-P. The network N comprises the system 1 for configuring a medical image processing workflow. The components of the system 1 may be spatially co-located with one or more of the workstations WS1, WS2, WS3 and/or with the development system DS. In one embodiment, one or more components of the system 1 are spatially co-located with the development system DS1 and one or more other components of the system 1 are spatially co-located with one or more of the workstations WS1, WS2, WS3.

Each of the workstations WS1, WS2, WS3 is located within a clinical environment, e.g. in a hospital. The workstation WS1 is located within a different clinical environment, e.g. in another hospital, than the workstation WS2. The development system DS is located external to each of the clinical environments of the workstations WS1, WS2, WS3 as illustrated by the dashed line.

Based on the type of training examples that is needed in order to train the machine learning algorithm and/or based on the expected variation in the processing results, the development system 1 may estimate the amount and type of data needed for the training. In order to obtain the data needed for the training, the development system DS contacts then workstations WS1, WS2, WS3. Each of the workstations WS1, WS2, WS3 may enable access for the development system DS to the processing results obtained by the corresponding workstation, e. g. by clinical users during their regular daily reading. Such results may be stored in a structured format in order to enable training of the machine learning algorithm.

In order to deal with the variability in the processing results due to subjective annotation, the medical image MI needs to be processed multiple times, at least twice, e.g. either by the same clinical user with a time interval in-between or by different clinical users. Therefore, at least one of the workstations WS1, WS2, WS3 and/or the development system DS may be configured for assigning/injecting a task regarding a planned second processing of the medical image MI into worklist of a clinical based on the processing precondition. The worklist may comprise tasks assigned to at least one of a given user or a group of users, a given workstation or a group of workstations, e.g. in the same clinical environment and/or in different clinical environments. A dedicated business/incentive model may be associated with the proposed approach in order to encourage clinical users to process additional cases which he or she would not have to deal with according to the regular clinical workflow.

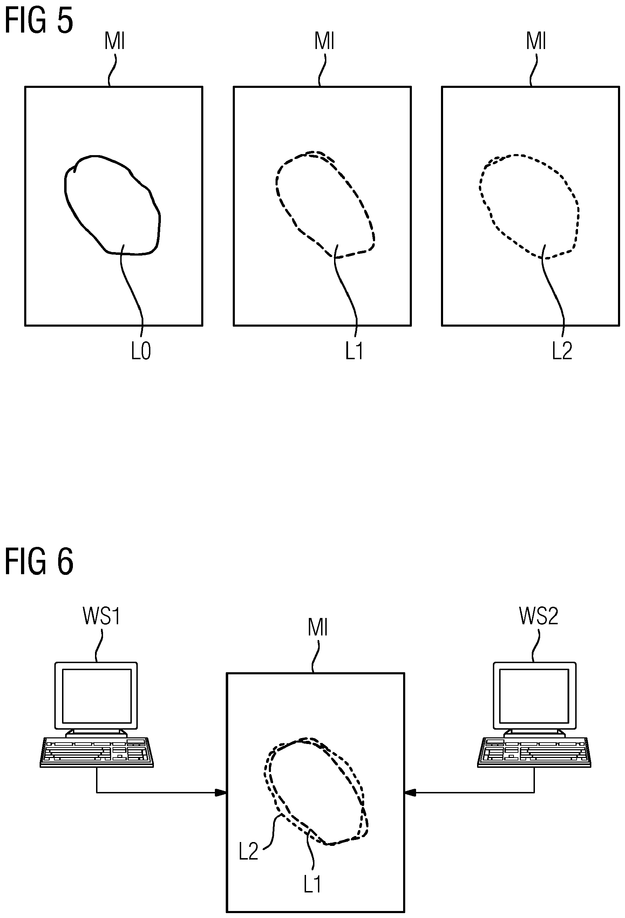

Fig. 5 shows examples of different processing results L1, L2 for the same medical image MI. The processing result L1 of the first processing of the medical image MI is a first segmentation mask of an anatomical structure L0 in the medical image MI. The processing result L2 of the planned second processing of the medical image MI is a second segmentation mask of the anatomical structure L0 in the medical image MI.

Fig. 6 shows examples of different processing results L1, L2 obtained using different workstations WS1, WS2. The processing result L1 has been obtained by a first user using workstation WS1. The processing result L2 has been obtained by a second user using workstation WS2. Based on the processing result L1 of the first processing of the medical image MI and the processing result L2 of the first processing of the medical image MI, the training precondition for the training of the machine learning algorithm may be adjusted. For example, if the variation in the processing results L1, L2 is found to be relatively high, a third processing of the medical image MI may be required. A processing precondition for a planned third processing of the medical image MI based on the training precondition, the first training pair and the second training pair may be determined.

The medical image MI may be thus processed multiple times and processing results may be made available to the development system DS. The second training pair comprising the medical image MI and a processing result L2 of the planned second processing of the medical image MI may comprise e.g. patient data as a part of the medical image MI and/or as a part of the processing result L2. The patient data may be transferred together with the second training pair to the development system for training of the machine learning algorithm. Alternatively, the second training pair may be processed by the workstation in order to remove sensitive information and/or patient data from the second training pair and subsequently transferred to the development system DS. Such an approach would help to avoid some of the aspects of data privacy, since patient data remains on the clinical site.

Therefore, the invention facilitates an improved collection of training data for machine learning, in particular an improved collection of clinical ground truth. The training data may be collected at clinical sites and the collection of the training data may be integrated into the daily clinical workflow. This is in contrast to dedicated sessions with experts, where annotation of data is performed. Such dedicated session often require huge organizational and financial effort and are therefore normally applied only to a limited number of cases as opposed to the proposed approach which can be used in a large scale and for a large number of cases, i.e. for big data applications.

## Claims

1. A method for configuring a medical dataset processing workflow, the method comprising:
- providing (PTN) a training precondition for a training of a machine learning algorithm,
- selecting (S1T) a first training pair for the training of the machine learning algorithm based on the training precondition, the first training pair comprising a medical dataset (MI) and a processing result of a first processing of the medical dataset (MI),
- determining (DRN) a processing precondition for a planned second processing of the medical dataset (MI) based on the training precondition and the first training pair,
- configuring (CW) a worklist of the medical dataset processing workflow based on the processing precondition, the configured worklist comprising a task regarding the planned second processing of the medical dataset (MI).

2. The method of claim 1, wherein the processing condition comprises
- a requirement regarding a time interval between the first processing of the medical dataset (MI) and the planned second processing of the medical dataset (MI) and/or
- a requirement regarding a user, to whom the task regarding the planned second processing of the medical dataset (MI) may be assigned.

3. The method of any one of the claims 1 to 2, wherein a graphical element (TA2) indicative of the task regarding the planned second processing is displayed in a graphical user interface (GUI) with regard to the processing condition.

4. The method of any one of the claims 1 to 3,
- wherein a user input in order to select the task regarding the planned second processing is received, and
- wherein a check with regard to the processing condition is performed upon receiving the user input.

5. The method of any one of the claims 1 to 4, wherein the planned second processing of the medical dataset (MI) is performed, thereby obtaining a second training pair for the training of the machine learning algorithm, the second training pair comprising the medical dataset (MI) and a processing result of the planned second processing of the medical dataset (MI).

6. The method of claim 5, wherein the machine learning algorithm is trained based on a set of training pairs, the set of training pairs comprising the first training pair and the second training pair.

7. The method of claim 5 or 6,
- wherein an uncertainty information is determined based on the first training pair and the second training pair and/or
- wherein the machine learning algorithm is trained based on the uncertainty information.

8. The method of any one of the claims 1 to 7,
- wherein the medical dataset processing workflow is a clinical workflow and/or is performed in a clinical environment and/or
- wherein the medical dataset processing workflow is a radiology reading workflow and/or a surgery planning workflow.

9. The method of any one of the claims 1 to 8,
- wherein the processing result of the first processing of the medical dataset (MI) is a first segmentation mask of an anatomical structure (LO) and the processing result of the planned second processing of the medical dataset (MI) is a second segmentation mask of the anatomical structure (LO) or
- wherein the processing result of the first processing of the medical dataset (MI) is a first centerline delineation of an anatomical structure (LO) and the processing result of the planned second processing of the medical dataset (MI) is a second centerline delineation of the anatomical structure (L0).

10. A system (1) for configuring a medical dataset processing workflow, the system comprising:
- a training-precondition-providing unit (PTN-U) configured for providing (PTN) a training precondition for a training of a machine learning algorithm,
- a first training-pair-selecting unit (S1T-U) configured for selecting (S1T) a first training pair for the training of the machine learning algorithm based on the training precondition, the first training pair comprising a medical dataset (MI) and a processing result of a first processing of the medical dataset (MI),
- a processing-precondition-determining unit (DRN-U) configured for determining (DRN) a processing precondition for a planned second processing of the medical dataset (MI) based on the training precondition and the first training pair,
- a worklist-configuring unit (CW-U) configured for configuring (CW) a worklist of the medical dataset processing workflow based on the processing precondition, the configured worklist comprising a task regarding the planned second processing of the medical dataset (MI).

11. The system (1) of claim 10, the system further comprising:
- a training unit (MLT-U) configured for the training of the machine learning algorithm and/or
- a medical dataset processing unit (MIP-U) configured for performing the planned second processing of the medical dataset (MI).

12. The system (1) of claim 11,
- wherein the medical dataset processing unit (MIP-U) is located inside a clinical environment and
- wherein the training unit (MLT-U) is located external to the clinical environment.

13. The system (1) of any one of the claims 10 to 12, configured for performing the method of any of claims 1 to 9.

14. A computer program product including a computer program, directly loadable into a memory device of a computer, including program sections for carrying out the method of any one of the claims 1 to 9 when the computer program is executed in said computer.

15. A computer-readable medium (30-M, DS-M) storing program sections, readable and executable by a computer, to carry out the method of any one of the claims 1 to 9 when the program sections are executed by the computer.
